# EUROPÄISCHE PATENTANMELDUNG

(11) **EP 1 921 151 A1**
(43) Veröffentlichungstag der Anmeldung: **14.05.2008**
(21) Anmeldenummer: 08075086.2
(22) Anmeldetag: 19.09.2001
(51) Int. Cl.: C12N 15/77, C12N 15/68

(54) **Verfahren zur Veränderung des Genoms von Corynebakterien**

(30) Priorität: 20.09.2000 DE 10046870
(62) Teilanmeldung aus: 01982319.4
(71) Anmelder: BASF SE, 67056 Ludwigshafen (DE)
(72) Erfinder: Pompejus, Markus, 67251 Freinsheim (DE); Schröder, Hartwig, 69226 Nussloch (DE); Kröger, Burkhard, 67117 Limburgerhof 67117 (DE); Zelder, Oskar, 67346 Speyer (DE)

(57) **Zusammenfassung**

Die Erfindung betrifft ein Verfahren zur Herstellung von Corynebakterien enthaltend eine oder mehrere geänderte genomische Sequenzen, wobei ein in Corynebakterien nicht replizierender Vektor verwendet wird, dessen Nukleinsäure von Corynebakterien nicht als fremd erkannt wird.

## Beschreibung

Die Erfindung betrifft ein neues Verfahren zur Veränderung des Genoms von Corynebakterien, Verwendung dieser Bakterien und neue Vektoren. Insbesondere betrifft die Erfindung ein Verfahren zur Veränderung von Corynebakterien mit Hilfe von in Corynebakterien nicht replizierbarer Vektoren.

Corynebacterium glutamicum ist ein gram-positives, aerobes Bakterium, das (wie auch andere Corynebakterien, d.h. Corynebacterium und Brevibacterium - Arten) in der Industrie für die Produktion einer Reihe von Feinchemikalien, und auch zum Abbau von Kohlenwasserstoffen und zur Oxidation von Terpenoiden verwendet wird (Zur Über sicht siehe z.B. Liebl (1992) "The Genus Corynebacterium", in: The Procaryotes, Volume II, Balows. A. et al., eds. Springer).

Aufgrund der Verfügbarkeit von Klonierungsvektoren zur Verwendung in Corynebakterien und Techniken zur genetischen Manipulation von C. glutamicum und verwandten Corynebacterium und Brevibacterium- Arten (siehe z.B. Yoshihama et al., J. Bacteriol. 162 (1985) 591-597; Katsumata et al., J. Bacteriol. 159 (1984) 306-311 und Santamaria et al. J. Gen. Microbiol. 130 (1984) 2237-2246) ist es möglich, diese Organismen genetisch zu verändern (bspw. durch Überexpression von Genen) um sie bspw. als Produzenten von einer oder mehreren Feinchemikalien besser und effizienter zu machen.

Die Verwendung von Plasmiden, die in Corynebakterien replizieren können ist dabei eine gut etablierte Technik, die dem Fachmann bekannt ist, breit angewendet wird und mehrfach in der Literatur dokumentiert ist (siehe z.B. Deb, J.K et al. (1999) FEMS Microbiol. Lett. 175, 11-20).

Es ist ebenfalls möglich. Corynebakterien dadurch genetisch zu verändern, dass die DNA-Sequenz des Genoms modifiziert wird. Es können DNA- Sequenzen in das Genom eingebracht werden (neu eingebracht und/oder vorhandene Sequenzen in weiteren Kopien eingebracht werden), es können auch DNA-Sequenzabschnitte aus dem Genom entfernt werden (z.B. Gene oder Teile von Genen), es können aber auch Sequenzaustausch (z.B. Basenaustausche) im Genom durchgeführt werden.

Die Veränderung des Genoms kann dadurch erreicht werden, dass DNA in die Zelle eingebracht wird, die vorzugsweise nicht in der Zelle repliziert und dass diese eingebrachte DNA mit genomischer Wirts-DNA rekombiniert und so die genomische DNA verändert. Die hierfür bekannten Methoden sind aber aufwendig und alle mit speziellen Problemen versehen (siehe z.B. van der Rest, M.E. et al. (1999) Appl. Microbiol. Biotechnol. 52, 541-545).

Ein bekanntes Verfahren basiert auf Konjugation (Schwarzer & Pühler (1991) Biotechnology 9, 84-87). Der Nachteil ist, dass spezielle mobilisierbare Plasmide verwendet werden müssen, die konjugativ von einem Donor-Stamm (in der Regel E. coli) zum Empfänger (bspw. Corynebacterium Arten) übertragen werden müssen. Diese Methode ist zudem sehr arbeitsaufwendig.

Die Nachteile der Konjugation sind der Grund, dass es auch zur Veränderung genomischer Sequenzen (und nicht nur zum Einbringen von frei replizierenden Plasmiden) vorteilhaft ist, statt der Konjugation die etablierte einfache Methode der Elektroporation (Liebl et al. (1989) FEMS Microbiol Lett. 65, 299-304) durchzuführen. Es wurde eine neue Methode beschrieben, die dies zwar ermöglicht (van der Rest, M.E. et al. (1999) Appl. Microbiol. Biotechnol. 52, 541-545), dafür aber andere Probleme hat. Die zu transformierenden Zellen werden bei sub-optimalen tiefen Temperaturen kultiviert, dem Wachstumsmedium werden spezielle Wachstums- beeinträchtigende Mediumszusätze zugegeben und die Zellen werden mit einem Hitzeschock behandelt.

Alle Methoden des DNA-Transfers in Corynebakterien haben als Problem das Wirtseigene Restriktionssystem von Corynebakterien, das als fremd erkannte DNA abbaut. Es gibt zahlreiche Ansätze in der Literatur, dieses Restriktionssystem zu umgehen, die aber alle spezifische Probleme haben.

Es gibt Versuche, DNA aus E. coli Stämmen einzusetzen, die Mutationen in den dam und dem Genen tragen (Ankri et al. (1996) Plasmid 35. 62-66). Dies führt zu DNA, die keine Dam und Dem Methylierung mehr trägt, aber weiterhin die E. coli- spezifische hsd Methylierung besitz. Diese DNA wird weiterhin von Corynebacterium als Fremd-DNA erkannt.

Eine Möglichkeit, Probleme mit dem Restriktionssystem zu umgehen ist es, Restriktionsdefiziente Mutanten zu isolieren (Liebl et al. (1989) FEMS Microbiol Lett. 65, 299-304). Der Nachteil ist aber, dass man aber auf solche speziellen Mutanten-Stämme beschränkt ist.

Ein anderer Weg ist die temporäre Ausschaltung des Restriktionssystems z.B. durch Hitzeschock. Sowohl bei Konjugation (Schwarzer & Pühler (1991) Biotechnology 9, 84-87) als auch bei Elektroporation (van der Rest, M.E. et al. (1999) Appl. Microbiol. Biotechnol. 52, 541-545) kann damit ein gewünschter Effekt erzielt werden. Nachteile sind die aufwendige Durchführung und der Effekt, dass durch den Hitzeschock nicht nur das Restriktionssystem sondern auch zahlreiche andere Zell-Prozesse beeinflusst werden. Generell hat die Hitzeschockantwort bei Bakterien als Reaktion auf den Hitzeschock eine Vielzahl von Konsequenzen für den Stoffwechsel der Zellen (siehe z.B. Gross, C.A. (1996), pp. 1382-1399 in Escherichia coli and Salmonella (Neidhart et al., eds.) ASM press, Washington).

Unter Corynebakterien im Sinne der Erfindung werden Corynebacterium- Arten. Brevibacterium- Arten und Mycobacterium- Arten verstanden. Bevorzugt sind Corynebacterium- Arten und Brevibacterium- Arten. Beispiele für Corynebacterium- Arten und Brevibacterium- Arten sind: Brevibacterium brevis, Brevibacterium lactofermentum, Corynebacterium ammoniagenes, Corynebacterium glutamicum, Corynebacterium diphtheriae, Corynebacterium lactofermentum. Beispiele für Mycobacterium- Arten sind: Mycobacterium tuberculosis. Mycobacterium leprae, Mycobacterium bovis.

Insbesondere sind folgende in der Tabelle angegebenen Stämme bevorzugt:

**Tabelle: Corynebacterium und Brevibacterium Stämme:**

| Genus | Species | ATCC | FERM | NRRL | CEC T | NCIMB | |
|---|---|---|---|---|---|---|---|
| Brevibacterium | Ammoniagenes | 21054 | | | | | |
| Brevibacterium | Ammoniagenes | 19350 | | | | | |
| Brevibacterium | Ammoniagenes | 19351 | | | | | |
| Brevibacterium | Ammoniagenes | 19352 | | | | | |
| Brevibacterium | Ammoniagenes | 19353 | | | | | |
| Brevibacterium | Ammoniagenes | 19354 | | | | | |
| Brevibacterium | Ammoniagenes | 19355 | | | | | |
| Brevibacterium | Ammoniagenes | 19356 | | | | | |
| Brevibacterium | Ammoniagenes | 21055 | | | | | |
| Brevibacterium | Ammoniagenes | 21077 | | | | | |
| Brevibacterium | Ammoniagenes | 21553 | | | | | |
| Brevibacterium | ammoniagenes | 21580 | | | | | |
| Brevibacterium | ammoniagenes | 39101 | | | | | |
| Brevibacterium | butanicum | 21196 | | | | | |
| Brevibacterium | divaricatum | 21792 | P928 | | | | |
| Brevibacterium | flavum | 21474 | | | | | |
| Brevibacterium | flavum | 21129 | | | | | |
| Brevibacterium | flavum | 21518 | | | | | |
| Brevibacterium | flavum | | | B11474 | | | |
| Brevibacterium | flavum | | | B11472 | | | |
| Brevibacterium | flavum | 21127 | | | | | |
| Brevibacterium | flavum | 21128 | | | | | |
| Brevibacterium | flavum | 21427 | | | | | |
| Brevibacterium | flavum | 21475 | | | | | |
| Brevibacterium | flavum | 21517 | | | | | |
| Brevibacterium | flavum | 21528 | | | | | |
| Brevibacterium | flavum | 21529 | | | | | |
| Brevibacterium | flavum | | | B11477 | | | |
| Brevibacterium | flavum | | | B11478 | | | |
| Brevibacterium | flavum | 21127 | | | | | |
| Brevibacterium | flavum | | | B11474 | | | |
| Brevibacterium | healii | 15527 | | | | | |
| Brevibacterium | ketoglutamicum | 21004 | | | | | |
| Brevibacterium | ketoglutamicum | 21089 | | | | | |
| Brevibacterium | ketosoreductum | 21914 | | | | | |
| Brevibacterium | lactofermentum | | | | 70 | | |
| Brevibacterium | lactofermentum | | | | 74 | | |
| Brevibacterium | lactofermentum | | | | 77 | | |
| Brevibacterium | lactofermentum | 21798 | | | | | |
| Brevibacterium | lactofermentum | 21799 | | | | | |
| Brevibacterium | lactofermentum | 21800 | | | | | |
| Brevibacterium | lactofermentum | 21801 | | | | | |
| Brevibacterium | lactofermentum | | | B11470 | | | |
| Brevibacterium | lactofermentum | | | B11471 | | | |
| Brevibacterium | lactofermentum | 21086 | | | | | |
| Brevibacterium | lactofermentum | 21420 | | | | | |
| Brevibacterium | lactofermentum | 21086 | | | | | |
| Brevibacterium | lactofennentum | 31269 | | | | | |
| Brevibacterium | linens | 9174 | | | | | |
| Brevibacterium | linens | 19391 | | | | | |
| Brevibacterium | linens | 8377 | | | | | |
| Brevibacterium | paraffinolyticum | | | | | 11160 | |
| Brevibacterium | spec. | | | | | | CBS 717.73 |
| Brevibacterium | spec. | | | | | | CBS 717.73 |
| Brevibacterium | spec. | 14604 | | | | | |
| Brevibacterium | spec. | 21860 | | | | | |
| Brevibacterium | spec. | 21864 | | | | | |
| Brevibacterium | spec. | 21865 | | | | | |
| Brevibacterium | spec. | 21866 | | | | | |
| Brevibacterium | spec. | 19240 | | | | | |
| Corynebacterium | acetoacidophilum | 21476 | | | | | |
| Corynebacterium | acetoacidophilum | 13870 | | | | | |
| Corynebacterium | acetoglutamicum | | | B11473 | | | |
| Corynebacterium | acetoglutamicum | | | B11475 | | | |
| Corynebacterium | acetoglutamicum | 15806 | | | | | |
| Corynebacterium | acetoglutamicum | 21491 | | | | | |
| Corynebacterium | acetoglutamicum | 31270 | | | | | |
| Corynebacterium | acetophilum | | | B3671 | | | |
| Corynebacterium | ammoniagenes | 6872 | | | | | NCTC 2399 |
| Corynebacterium | ammoniagenes | 15511 | | | | | |
| Corynebacterium | fujiokense | 21496 | | | | | |
| Corynebacterium | glutamicum | 14067 | | | | | |
| Corynebacterium | glutamicum | 39137 | | | | | |
| Corynebacterium | glutamicum | 21254 | | | | | |
| Corynebacterium | glutamicum | 21255 | | | | | |
| Corynebacterium | glutamicum | 31830 | | | | | |
| Corynebacterium | glutamicum | 13032 | | | | | |
| Corynebacterium | glutamicum | 14305 | | | | | |
| Corynebacterium | glutamicum | 15455 | | | | | |
| Corynebacterium | glutamicum | 13058 | | | | | |
| Corynebacterium | glutamicum | 13059 | | | | | |
| Corynebacterium | glutamicum | 13060 | | | | | |
| Corynebacterium | glutamicum | 21492 | | | | | |
| Corynebacterium | glutamicum | 21513 | | | | | |
| Corynebacterium | glutamicum | 21526 | | | | | |
| Corynebacterium | glutamicum | 21543 | | | | | |
| Corynebacterium | glutamicum | 13287 | | | | | |
| Corynebacterium | glutamicum | 21851 | | | | | |
| Corynebacterium | glutamicum | 21253 | | | | | |
| Corynebacterium | glutamicum | 21514 | | | | | |
| Corynebacterium | glutamicum | 21516 | | | | | |
| Corynebacterium | glutamicum | 21299 | | | | | |
| Corynebacterium | glutamicum | 21300 | | | | | |
| Corynebacterium | glutamicum | 39684 | | | | | |
| Corynebacterium | glutamicum | 21488 | | | | | |
| Corynebacterium | glutamicum | 21649 | | | | | |
| Corynebacterium | glutamicum | 21650 | | | | | |
| Corynebacterium | glutamicum | 19223 | | | | | |
| Corynebacterium | glutamicum | 13869 | | | | | |
| Corynebacterium | glutamicum | 21157 | | | | | |
| Corynebacterium | glutamicum | 21158 | | | | | |
| Corynebacterium | glutamicum | 21159 | | | | | |
| Corynebacterium | glutamicum | 21355 | | | | | |
| Corynebacterium | glutamicum | 31808 | | | | | |
| Corynebacterium | glutamicum | 21674 | | | | | |
| Corynebacterium | glutamicum | 21562 | | | | | |
| Corynebacterium | glutamicum | 21563 | | | | | |
| Corynebacterium | glutamicum | 21564 | | | | | |
| Corynebacterium | glutamicum | 21565 | | | | | |
| Corynebacterium | glutamicum | 21566 | | | | | |
| Corynebacterium | glutamicum | 21567 | | | | | |
| Corynebacterium | glutamicum | 21568 | | | | | |
| Corynebacterium | glutamicum | 21569 | | | | | |
| Corynebacterium | glutamicum | 21570 | | | | | |
| Corynebacterium | glutamicum | 21571 | | | | | |
| Corynebacterium | glutamicum | 21572 | | | | | |
| Corynebacterium | glutamicum | 21573 | | | | | |
| Corynebacterium | glutamicum | 21579 | | | | | |
| Corynebacterium | glutamicum | 19049 | | | | | |
| Corynebacterium | glutamicum | 19050 | | | | | |
| Corynebacterium | glutamicum | 19051 | | | | | |
| Corynebacterium | glutamicum | 19052 | | | | | |
| Corynebacterium | glutamicum | 19053 | | | | | |
| Corynebacterium | glutamicum | 19054 | | | | | |
| Corynebacterium | glutamicum | 19055 | | | | | |
| Corynebacterium | glutamicum | 19056 | | | | | |
| Corynebacterium | glutamicum | 19057 | | | | | |
| Corynebacterium | glutamicum | 19058 | | | | | |
| Corynebacterium | glutamicum | 19059 | | | | | |
| Corynebacterium | glutamicum | 19060 | | | | | |
| Corynebacterium | glutamicum | 19185 | | | | | |
| Corynebacterium | glutamicum | 13286 | | | | | |
| Corynebacterium | glutamicum | 21515 | | | | | |
| Corynebacterium | glutamicum | 21527 | | | | | |
| Corynebacterium | glutamicum | 21544 | | | | | |
| Corynebacterium | glutamicum | 21492 | | | | | |
| Corynebacterium | glutamicum | | | B8183 | | | |
| Corynebacterium | glutamicum | | | B8182 | | | |
| Corynebacterium | glutamicum | | | B12416 | | | |
| Corynebacterium | glutsmicum | | | B12417 | | | |
| Corynebacterium | glutamicum | | | B12418 | | | |
| Corynebacterium | glutamicum | | | B11476 | | | |
| Corynebacterium | glutamicum | 21608 | | | | | |
| Corynebacterium | lilium | | P973 | | | | |
| Corynebacterium | nitrilophilus | 21419 | | | | 11594 | |
| Corynebacterium | spec. | | P4445 | | | | |
| Corynebacterium | spec. | | P4446 | | | | |
| Corynebacterium | spec. | 31088 | | | | | |
| Corynebacterium | spec. | 31089 | | | | | |
| Corynebacterium | spec. | 31090 | | | | | |
| Corynebacterium | spec. | 31090 | | | | | |
| Corynebacterium | spec. | 31090 | | | | | |
| Corynebacterium | spec. | 15954 | | | | | DSMZ 20145 |
| Corynebacterium | spec. | 21857 | | | | | |
| Corynebacterium | spec. | 21862 | | | | | |
| Corynebacterium | spec. | 21863 | | | | | |

| | | | | | | | |
|---|---|---|---|---|---|---|---|
| ATCC: American Type Culture Collection, Rockville, MD, USA FERM: Fermentation Research Institute. Chiba, Japan NRRL: ARS Culture Collection, Northem Regional Research Laboratory, Peoria, IL, USA CECT: Coleccion Espanola de Cultivos Tipo, Valencia, Spain NCIMB: National Collection of Industrial and Marine Bacteria Ltd., Aberdeen, UK CBS: Centraalbureau voor Schimmelcultures, Baarn, NL NCTC: National Collection of Type Cultures, London, UK DSMZ: Deutsche Sammlung von Mikroorganismen und Zellkulturen, Braunschweig, Germany | | | | | | | |

Die Erfindung offenbart eine neue und einfache Methode zur Veränderung von genomischen Sequenzen in Corynebakterien. Dies können genomische Integrationen von Nukleinsäuremolekülen (bspw. komplette Gene), Disruptionen (bspw. Deletionen oder integrative Disruptionen) und Sequenzveränderungen (bspw. einfache oder mehrfache Punktmutationen, komplette Gen-Austausche) sein. Die oben beschriebenen Probleme treten hierbei nicht auf. Die erfindungsgemäße Methode ist nicht abhängig von der Verwendung spezieller Empfängerstämme und bedarf nur der üblicherweise verwendeten Methoden der Kultivierung der Zellen und der Transformation.

Von Schäfer et al. (Gene 203, 1997, 93-101) wurde das cgIIM Gen von Corynebacterium glutamicum beschrieben. Dieses Gen kodiert eine DNA-Methyltransferase. Zudem wird eine Methode beschrieben, bei Verwendung von replikativen Plasmiden die Ausbeute an C. glutamicum Transformanten mit Hilfe des cgIIM Gens zu erhöhen.

Es wurde gefunden, dass man Methyltransferasen insbesondere das cgIIM Gen auch dazu verwenden kann. DNA in das Genom von Corynebacterium glutamicum zu integrieren um bspw. Gene im Genom zu disruptieren oder zu überexprimieren. Dies ist auch möglich mit anderen das Corynebakterien-spezifische Methylierungsmuster einführenden Methyltransferasen. Hierfür wird ein in dem zu transformierenden Corynebakterium nicht replizierbarer Vektor verwendet. Unter einem nicht replizierbaren Vektor wird eine DNA verstanden, die nicht frei in Corynebakterien replizieren kann. Es ist möglich dass diese DNA in anderen Bakterien frei replizieren kann, wenn sie bspw. einen entsprechenden origin of replication trägt. Es ist aber auch möglich, dass diese DNA auch in anderen Bakterien nicht replizieren kann, wenn bspw. eine lineare DNA eingesetzt wird.

Das erfindungsgemäße Verfahren beruht auf einer direkten Transformation von C. glutamicum (z.B. durch Eiektroporation), ohne daß spezielle Methoden der Anzucht der zu transformierenden Zellen oder besondere Verfahren bei der Transformation (wie Hitzeschock etc.) verwendet werden müssen.

Die Transformation kann auch mit Zugabe von Restriktionsendonukleasen (wie in DE19823834 beschrieben) durchgeführt werden.

Der Vorteil des erfindungsgemäßen Verfahrens ist, dass die eingebrachte DNA nicht als Fremd-DNA erkannt wird und sie durch das Restriktionssystem deshalb nicht abgebaut wird.

Ein weiterer Vorteil des erfindungsgemäßen Verfahrens ist, daß keine Konjugation durchgeführt werden muß - das vermindert den Arbeitsaufwand beträchtlich und ermöglicht verbesserte Flexibilität bei der Wahl der eingesetzten Plasmide.

Ein weiterer Vorteil ist, dass keine speziellen Corynebakterien-Stämme eingesetzt werden müssen und dass keine spezielle Behandlung der zu transformierenden Stämme notwendig ist, insbesondere ist kein Hitzeschock notwendig. Für experimenteile Details siehe Beispiel.

Die so erzeugten Mutanten können dann zur Herstellung von Feinchemikalien verwendet werden oder im Falle von C. diphtheriae für die Herstellung z.B. von Impfstoffen mit abgeschwächten oder nicht-pathogenen Erregern. Unter Feinchemikalien werden verstanden: organische Säuren, sowohl proteinogene als auch nicht-proteinogene Aminosäuren, Nukleotide und Nukleoside, Lipide und Fettsäuren, Diole, Kohlehydrate, aromatische Verbindungen, Vitamine und Cofaktoren sowie Enzyme.

Der Begriff "Feinchemikalie" ist im Fachgebiet bekannt und beinhaltet Moleküle, die von einem Organismus produziert werden und in verschiedenen Industriezweigen Anwendungen finden, wie bspw., jedoch nicht beschränkt auf die pharmazeutische Industrie, die Landwirtschafts-, und Kosmetik-Industrie. Diese Verbindungen umfassen organische Säuren, wie Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlenhydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1. bis 3. Sept. 1994 in Penang, Malaysia, AOCS Press (1995)), Enzyme Polyketide (Cane et al. (1998) Science 282: 63-68), und sämtliche anderen von Gutcho (1983) in Chemicals by Fermentation, Noyes Data Corporation, ISBN: 0818805086 und den darin angegebenen Literaturstellen, beschriebenen Chemikalien. Der Metabolismus und die Verwendungen bestimmter Feinchemikalien sind nachstehend weiter erläutert.

### A. Aminosäure-Metabolismus und Verwendungen

Die Aminosäuren umfassen die grundlegenden Struktureinheiten sämtlicher Proteine und sind somit für die normalen Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ullmann's Encyclopedia of industrial Chemistry, Bd. A2, S. 57-97 VCH: Weinheim (1985)). Die Aminosäuren können in der D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L. Biochemistry. 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität ihrer Biosynthesen mit der Ernährung aufgenommen werden müssen, werden durch einfache Biosynthesewege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihre Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine Wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Enryclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation, s. Umbarger. H.E. (1978) Ann. Rev. Biochem. 47: 533-606). Glutamat wird durch reduktive Aminierung von - Ketoglutarat, einem Zwischenprodukt im Citronensäuro-Zyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten- -Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet. Tryptophan wird ebenfalls aus diesen beiden Ausgangsmoleküle produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf der Zelle übersteigt, können nicht gespeichert werden, und werden stattdessen abgebaut, so daß Zwischenprodukte für die Haupt-Stoffwechselwege der Zelle bereitgestellt werden (für einen Überblick siehe Stryor, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäure-Produktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### B. Vitamine, Cofaktoren und Nutrazeutika-Metabolismus sowie Verwendungen

Vitamine Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullmann's Encyclopedia of industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullmann's Encyclopedia of Industrial Chemistry, "Vitamins", Bd. A27, S. 443-613. VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X. 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B6" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)- -alanin) kann entweder durch chemische Synthese oder durch Fermentation hergestellt werden. Die letzten Schritte bei der Pantothenat-Biosynthese bestehen aus der ATP-getriebenen Kondensation von -Alanin und Pantoinsäure. Die für die Biosyntheseschritte für die Umwandlung in Pantoinsäure, in -Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den metabolischen Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukieotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien ebenfalls durch großangelegte Anzucht von Mikroorganismen produziert worden sind, wie Riboflavin, Vitamin B₆, Pantothenat und Biotin. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### C. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteil der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisation zur Bildung von Nukleinsäuremolekülen ist es möglich, die RNA- und DNA-Synthese zu hemmen; wird diese Aktivität zielgerichtet bei kanzerogenen Zellen gehemmt, läßt sich die Teilungs- und Replikations-Fähigkeit von Tumorzellen hemmen.

Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.l. und Lyons, S.D. (1990) "Potent inhibitors of de novo pyrimidine and purine biosynthesis as chemotherapeutic agents", Med. Res. Reviews 10: 505-548). Untersuchungen an Enzymen, die am Purin- und Pyrimidin-Metabolismus beteiligt sind, haben sich auf die Entwicklung neuer Medikamente konzentriert, die bspw. als Immunsuppressionsmittel oder Antiproliferantien verwendet werden können (Smith, J.L. "Enzymes in Nucleotide Synthesis" Curr. Opin. Struct. Biol. 5 (1995) 752-757; Biochem. Soc. Transact. 23 (1995) 877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, werden gewöhnlich als Geschmacksverstärker vervvendet (bspw. IMP oder GMP) oder für viele medizinische Anwendungen (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden. Herbiziden und Insektiziden entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular biology, Bd. 42, Academic Press.S. 259-287; und Michal, G. (1999) "Nucleotides and Nucleosides": Kap. 8 in : Biochemkal Pathways: An Atlas of Biochemistry and Molecular Biology, Wiley, New York). Der Purin-Metabolismus, das Objekt intesiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden über eine Reihe von Schritten über die Zwischenverbindung Inosin-5'-phosphat (IMP) aus Ribose-5-phosphat synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt, Nach der Phosphorylierung können diese Moleküle an der DNA-Synthese teilnehmen.

### D. Trehalose-Metabolismus und Verwendungen

Trehalose besteht aus zwei Glucosemolekülen, die über , -1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist. S. Trends Biotech. 16 (1998) 460-467; Paiva, C.L.A. und Panek, A.D. Biotech Ann. Rev. 2 (1996) 293-314; und Shiosaka, M. J. Japan 172 (1997) 97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

Dieses Vorgehen kann in analoger Weise auch mit anderen Bakterien durchgeführt werden.

### Beispiel:

Man kann einen beliebigen Sequenzabschnitt des ddh-Gens von C. glutamicum (ishino et al.(1987) Nucleic Acids Res. 15, 3917), insbesondere ein Fragment im 5'-terminalen Bereich der kodierenden Region mit bekannten Methoden per PCR amplifizieren und das resultierende PCR-Produkt in pSL18 (Kim, Y. H. & H.-S. Lee (1996) J. Microbiol. Biotechnol. 6,315-320) klonieren und so den Vektor pSL18 ddh erhalten. Man kann dafür auch andere Vektoren mit einem für C. glutamicum geeigneten Markergen verwenden. Die Vorgehensweise ist dem Fachmann geläufig.

Man kann das cgIIM-Gen in einem geeigneten E. coli Stamm (McrBC-defizient (alternative Bezeichnung hsdRM defizient) wie z.B. NM522 oder HB101) auf unterschiedliche Weise exprimieren, sowohl als genomische Kopie als auch auf Plasmiden. Eine Methode beruht auf der Verwendung des Plasmides pTc15AcgIIM. Das Plasmid pTc15AcgIIM umfasst den Replikationsursprung des Plasmides p15A (Selzer et al. (1983) Cell 32, 119-129), ein Gen für Resistenz gegen Tetracyclin (Genbank Acc. No. J01749) und das cgIIM-Gen (Schäfer et al. (1997) Gene 203,93-101). E. coli Stämme, die pTc15AcgIIM tragen, haben DNA die das cgIIM Methylierungsmuster trägt. Entsprechend sind die pSL18-Derivate (wie pSL 18 ddh, siehe oben) ebenfalls "cgIIMmethyliert".

Man kann die Plasmid-DNA des Stammes NM522(pTc15AcgIIM/pSL18 ddh) nach üblichen Methoden (Sambrook. J. et al. (1989) "Molecular Cloning: A Laboratory Manual". Cold Spring Harbor Laboratory Press oder Ausubel, F.M. et al. (1994) "Current Protocols in Molecular Biology", John Wiley & Sons) präparieren und diese DNA zur Elektroporation von C. glutamicum (Liebl et al. (1989) FEMS Microbiol. Lett. 65, 299-304) einsetzen. Dabei kann C. glutamicum ATCC13032 verwendet werden, es können aber auch andere Corynebakterien verwendet werden.

Plasmid pSL18 ddh gewonnen aus einem E. coli Stamm ohne pTc15AcgllM führte bei keinem unserer Versuche zu Transformanten nach Elektroporation. Im Gegensatz dazu, führte pSL18 ddh gewonnen aus einen pTc15AcgIIM-tragenden E. coli Stamm dazu, daß Transformanten durch Elektroporation gewonnen werden konnten. Diese Transformanten warlen Klone, bei denen das ddh-Gen deaktiviert wurde, wie bspw. durch fehlende Ddh-Aktivität gezeigt werden konnte. Ddh-Aktivität kann nach bekannten Methoden (siehe z.B. Misono et al. (1986) Agric.Bioi.Chem. 50, 1329-1330) gemessen werden.

## Patentansprüche

1. Verfahren zur Herstellung von Corynebakterien enthaltend eine oder mehrere geänderte genomische Sequenzen, wobei ein in Corynebakterien nicht replizierender Vektor verwendet wird, dessen Nukleinsäure von Corynebakterien nicht als fremd erkannt wird.

2. Verfahren nach Anspruch 1, wobei der Vektor das corynebakterielle DNA- Methylierungsmuster trägt.

3. Verfahren nach Anspruch 2, wobei das Methylierungsmuster durch eine Methyltransferase erhältlich ist.

4. Verfahren nach einem der Ansprüche 1 bis 3, wobei es sich bei den Corynebakterien um Corynebacterium glutamicum, Corynebacterium ammoniagenes, Corynebacterium diphtheriae, Corynebacterium lactofermentum, Brevibacterium lactofermentum, Brevibacterium brevis handelt.

5. Verfahren nach einem der Ansprüche 1 bis 4, wobei es sich bei den veränderten genomischen Sequenzen um eine oder mehrere Punktmutationen, eine oder mehrere Disruptionen, Einbringen eines oder mehrerer im Organismus vorhandener oder fremder Gene, handelt.

6. Verfahren zur Herstellung von Feinchemikalien, wobei ein nach einem der in den Ansprüchen 1 bis 5 beanspruchten Verfahren hergestellter Mikroorganismus zur Produktion der Feinchemikalie verwendet wird.

7. Verfahren nach Anspruch 6. wobei die Feinchemikalie eine natürlich vorkommende Aminosäure, insbesondere Lysin, Threonin, Glutamat oder Methionin ist, oder ein Vitamin, insbesondere Riboflavin oder Pantothensäure ist.

8. Verfahren nach einem der Ansprüche 2 bis 7, wobei das Methylierungsmuster durch die Methyltransferase cgIIM erhältlich ist.

9. Nicht in Corynebakterien replizierender Vektor mit einem Corynebakterienspezifischen Methylierungsmuster.

10. Vektor nach Anspruch 9 mit einem Methylierungsmuster erhältlich durch eine Methyltransferase, insbesondere cgIIM.
